# EUROPEAN PATENT APPLICATION

(11) **EP 4 520 327 A1**
(43) Date of publication of application: **12.03.2025**
(21) Application number: 24192750.8
(22) Date of filing: 03.08.2024
(51) Int. Cl.: A61K 31/435, A61K 31/4745, A61K 33/06, A61K 33/26, A61P 31/12, A61P 31/16, A61P 31/22

(54) **COMPOSITION FOR USE IN TREATING LONG COVID**

(30) Priority: 11.09.2023 US 202318244569
(71) Applicant: Kirschner, Ellen, 63150 Heusenstamm (DE)
(72) Inventor: Kirschner, Ellen, 63150 Heusenstamm (DE)
(74) Representative: Alatis

(57) **Abstract**

Compositions and methods of use for treatment of Long Covid, or other post-viral or other post-infection conditions that result in a toxin induced thickened blood pulp condition (e.g., including but not limited to influenza, zika, herpes, or epstein-barr viruses). The treatment composition includes C₂₉H₄₀N₂O₄; C₉H₂N₂; and/or C₂₈H₃₈N₂O₄; and optionally a source of iron. A filler (e.g., CaCO₃) or pharmaceutically acceptable carrier may be provided. In the treatment method, treatment composition is administered to a patient, specifically because of the patient's exhibition of symptoms of Long Covid (or other contemplated post-viral or other post-infection condition). The treatment composition can be administered as a single dose. The components thereof have a molecular and/or particle size sufficiently small so as to not induce vomiting, and the single dose administration to the patient is sufficient so that the patient's Long Covid symptoms are relieved within a period of about 10 days.

## Description

### CROSS-REFERENCE TO RELATED APPLICATIONS

This application claims priority to U.S. Patent Application Serial No. 18/244,569, filed September 11, 2023, which is hereby incorporated by reference in its entirety.

### BACKGROUND OF THE INVENTION

### 1. The Field of the Invention

The present invention relates to formulations and methods of treatment useful in preventing and treating Long Covid and other similar post-viral infection conditions.

### 2. Description of Related Art

Some who have been infected with the virus that causes COVID-19 can experience long-term lingering effects from such infection, known as Long Covid, where signs, symptoms and conditions continue or develop after an initial COVID-19 infection, even after the initial COVID-19 infection may have otherwise been resolved.

Such conditions can include a variety of ongoing health problems, and can last weeks, months or even years. For example, under normal circumstances, someone infected with COVID-19 who recovers will do so within a few days to a few weeks after initial infection. Even after initial recovery, Applicant has found that there are toxins resulting from metabolism of the Covid virus that remain in the body. In other words, the titers resulting from the metabolism of the Covid virus remain in the body. Where symptoms persist, or return, e.g., after 4 weeks or more, such a patient may be suffering from Long Covid.

Individuals with Long Covid may exhibit a wide variety of symptoms, examples of which include, but are not limited to, tiredness or fatigue, recurring fever, shortness of breath, cough, chest pain, heart palpitations, difficulty thinking clearly, headache, difficulty sleeping, dizziness, "pins and needles" sensations in the fingers, hands, and elsewhere, lingering change in the ability to smell or taste, diarrhea, stomach pain, joint or muscle pain, rash, changes in menstrual cycle, etc.

Such symptoms often cannot be explained, managed, or treated, using conventional treatments. For example, with Long Covid, conventional clinical evaluations and routine blood tests, chest x-rays, electrocardiograms and the results of other routine testing may appear relatively normal. This can make treatment of such a condition difficult.

It would be beneficial to provide a simple treatment that would prevent the development of Long Covid, and/or relieve and treat symptoms of Long Covid, allowing persons suffering from such to regain their pre-COVID health and energy, relatively quickly. It would be an additional advantage, if such treatment could also be used for other, similar post-viral infection conditions of the blood. The active substance disclosed for use herein is not a drug against Long Covid, but is key to the successful treatment of Long Covid.

### BRIEF SUMMARY OF THE INVENTION

Applicant has found that symptoms associated with Long Covid are preceded by decomposition of red blood cells (erythrocytes) within the patient. Individuals of all kinds are affected worldwide, including children, youth, adults, the elderly, and especially athletes with a strongly activated circulatory system, who have occasionally suffered surprisingly rapid cardiac arrest with little or no warning. Red blood cells in the acute phase of the infection, that have been attacked by residual toxins at a metabolic product after a patient's initial recovery from a COVID infection are degraded and decomposed as a metabolic product, reduced to an oxygenless "pulp" consistency, resulting in thickened sluggish blood, with an increased risk of dangerous blood clots. Such red blood cells that have been reduced to such a pulp cannot effectively carry oxygen. Any organ that is reached by this pulp is affected, due to lack of oxygen.

Applicant theorizes that from the first moment of infection with the COVID-19 virus, red blood cells are affected, and decomposed by such a toxin associated with COVID infection. In this metabolic process, alkaloids are formed, which further break down the red blood cells. Even after the initial COVID infection has passed (e.g., the individual no longer tests positive for COVID-19), these alkaloid toxins remain present in the blood cells, and in the nucleus and mitochondria, and cannot be readily eliminated from the body when relying on the body's natural immune system alone.

Available treatment options for Long Covid have not shown lasting success. For example, resuscitation after a heart attack that occurs in a patient suffering from Long Covid often fails because this mucus-like pulp is not calcification, but is dead and decomposed red blood cell pulp. Even a blood transfusion provides only temporary relief, which lasts only for a few weeks, as not all blood can be removed, and the alkaloid toxins are still present within the body, and continue to degrade replaced or newly produced red blood cells. What is needed is a way to neutralize and flush the alkaloid toxins from the body.

Applicant discovered the presently described treatment, as a result of believing (whether actually or not) she had ingested powdered dishwashing detergent in her coffee, by mistake. She administered a poison control treatment antigen, and found that such treatment surprisingly resulted in clearance of her Long Covid symptoms within a few days. Additional testing on dozens, even hundreds of additional patients suffering from Long Covid has confirmed to the Applicant that the treatment is in fact highly effective in resolving symptoms associated with Long Covid, with application of a single treatment dose of treatment, with neutralization of toxins/titers within about 30 minutes, and symptom resolution within a period of no more than about 10 days.

Adjustment of the initial treatment composition used by Applicant, and its administration to numerous others who have suffered from Long Covid symptoms has surprisingly shown that administration of such a treatment composition results in clearance of the Long Covid symptoms in nearly every individual so treated. A single treatment is sufficient to offer permanent relief, ending the symptoms associated with Long Covid. Where the Long Covid has affected other organs and systems, additional medications and treatments specific to the rehabilitation of such may be needed, e.g., in combination with the presently described treatment composition.

In one aspect, the present invention is directed to a treatment composition for use in treatment of Long Covid. The same treatment composition may be useful in treating other similar post-viral or post-bacterial infection conditions, affecting the blood system, that result in a toxin induced thickened blood pulp condition, or similar hematoxin conditions. In an embodiment, the treatment composition comprises a combination of alkaloid components that are able to neutralize the COVID alkaloid toxin, allowing the body's natural immune system to then flush it from the body, relatively quickly. With such toxins removed, any affected organs can be treated with appropriate Long Covid medications, for recovery of such organs (e.g., respiratory organs, CNS, lymph system, cardiovascular system, etc.) By way of example, the treatment composition may include C₂₉H₄₀N₂O₄, C₉H₂N₂, and/or C₂₈H₃₈N₂O₄. In an embodiment, all 3 such alkaloid components are included. Additional components, particularly a source of iron (e.g., FePO₄) may also be present, e.g., to stabilize non-poisoned erythrocytes, and aiding in regaining and boosting lost oxygen transport capacity. A filler, such as calcium carbonate, or another pharmaceutically acceptable carrier may also be present.

Another aspect of the invention relates to a method for treatment of Long Covid, or other post-viral or similar post-infection conditions that result in a toxin induced thickened blood pulp condition, the method comprising providing a treatment composition comprising: C₂₉H₄₀N₂O₄; C₉H₂N₂; and/or C₂₈H₃₈N₂O₄; and optionally a source of iron; and administering the treatment composition to a patient, the patient being specifically selected for administering the treatment composition thereto because of their exhibiting symptoms of Long Covid, or having recently recovered from an initial COVID-19 infection, or other post-viral or other post-infection condition.

Another aspect of the invention relates to a method for treatment of Long Covid, the method comprising providing a treatment composition comprising C₂₉H₄₀N₂O₄; C₉H₂N₂; C₂₈H₃₈N₂O₄; and optionally a source of iron; and administering the treatment composition to a patient, selected because of the patient's exhibition of symptoms of Long Covid, or having recently recovered from an initial COVID-19 infection (e.g., in order to prevent onset of Long Covid).

Another aspect of the invention relates to use of a treatment composition in a drug or other treatment composition, for treatment of Long Covid or other post-infection conditions, wherein the composition comprises C₂₉H₄₀N₂O₄; C₉H₂N₂; and/or C₂₈H₃₈N₂O₄.

Another aspect of the invention relates to use of a treatment composition to interfere with the metabolism of COVID-19 or other viruses, wherein the composition comprises C₂₉H₄₀N₂O₄; C₉H₂N₂; and/or C₂₈H₃₈N₂O₄.

In any of the described embodiments, the treatment composition may further compriseCaCO₃ or another filler or pharmaceutically acceptable carrier.

In any of the described embodiments, the source of iron may comprise FePO₄ or another suitable iron salt.

In any of the described embodiments, the composition can be administered in a single dose, wherein the single dose includes about 1/750,000 mg of C₂₉H₄₀N₂O₄, about 1/90,000 mg of C₉H₂N₂, about 1/750,000 mg of C₂₈H₃₈N₂O₄, about 2.1 mg of the source of iron, and about 4.1 mg of CaCO₃. More generally speaking, the C₂₉H₄₀N₂O₄ may be present in an amount from about 1/300,000 mg to about 1/750,000 mg, the C₂₈H₃₈N₂O₄ may be present in an amount from about 1/300,000 mg to about 1/750,000 mg, the C₉H₂N₂ may be present in an amount from about 1/60,000 mg to about 1/250,000 mg, the source of iron may be present in an amount from about 1 to about 5 mg, and the CaCO₃ may be present in an amount from about 1 to about 10 mg.

In addition to use to treat Long Covid, in any of the described embodiments, the treatment composition can be used to treat other post viral infection conditions, e.g., associated with influenza, zika, herpes, epstein-barr viruses, and/or other blood decomposing bacterial metabolites.

In any of the described embodiments, the treatment composition can be administered as a single dose, where the components thereof have a molecular and/or particle size sufficiently small so as to not induce vomiting, so as to be sufficiently small to penetrate the cell membrane, and the single dose administration to the patient is sufficient so that the patient's Long Covid symptoms are relieved within a period of 10 days.

It is important to recognize that the present compositions are not a cure for Long Covid, but are key to treatment of Long Covid. For example, it is believed that 1) the virus generates a metabolic product during its decomposition; 2) this metabolite, a cell decomposition alkaloid, is so finely molecular that it crosses the cell membrane; 3) in particular, it enters cells with mitochondria; 4) different cell disruption alkaloids in the mitochondria act on different tissue structures in the body - the cell destroying toxin of the coronavirus acts on the blood cells; 5) only a drug which is also of fine molecular weight can neutralize the cell disruption alkaloid in the mitochondria; 6) by stopping cell decomposition, the immune system can now work together with any needed Long Covid drugs, to achieve recovery within the patient.

By way of example and based on theory, in order for the presently contemplated active ingredients to be effective in the cells, they must be very small so that they can pass through the cell membrane. For example, it is believed that the cell membrane has correspondingly small openings that only very fine molecular substances can penetrate, and have an effect there. The Covid-19 toxin believed to be responsible for erythrocyte decomposition is similarly of a very small molecular size. Such toxins can therefore only be neutralized with another toxin (e.g., the presently described alkaloids). The metabolic toxins resulting from the decomposition of viruses and bacteria are so small that they can pass through the cell membrane, such that the presently contemplated active ingredient(s) have to be beaten or otherwise rendered so small that they can also pass through the cell membrane and neutralize the toxins. Applicant's formulation "ELENANT" includes active ingredient(s) that neutralize cell toxins that break down blood cells.

Further features and advantages of the present invention will become apparent to those of ordinary skill in the art in view of the detailed description of preferred embodiments below.

### DETAILED DESCRIPTION

### I. Definitions

Before describing the present invention in detail, it is to be understood that this invention is not limited to particularly exemplified systems or process parameters that may, of course, vary. It is also to be understood that the terminology used herein is for the purpose of describing particular embodiments of the invention only, and is not intended to limit the scope of the invention in any manner.

All publications, patents and patent applications cited herein, whether supra or infra, are hereby incorporated by reference in their entirety to the same extent as if each individual publication, patent or patent application was specifically and individually indicated to be incorporated by reference.

The term "comprising," which is synonymous with "including," "containing," or "characterized by," is inclusive or open-ended and does not exclude additional, unrecited elements or method steps.

The term "consisting essentially of" limits the scope of a claim to the specified materials or steps "and those that do not materially affect the basic and novel characteristic(s)" of the claimed invention.

The term "consisting of" as used herein, excludes any element, step, or ingredient not specified in the claim.

It must be noted that, as used in this specification and the appended claims, the singular forms "a", "an" and "the" include plural referents unless the content clearly dictates otherwise. Thus, for example, reference to an "alkaloid" includes one, two or more such alkaloids.

Numbers, percentages, ratios, or other values stated herein may include that value, and also other values that are about or approximately the stated value, as would be appreciated by one of ordinary skill in the art. As such, all values herein are understood to be modified by the term "about". A stated value should therefore be interpreted broadly enough to encompass values that are at least close enough to the stated value to perform a desired function or achieve a desired result, and/or values that round to the stated value. The stated values include at least the variation to be expected in a typical manufacturing process, and may include values that are within 10%, within 5%, within 1%, etc. of a stated value.

Some ranges may be disclosed herein. Additional ranges may be defined between any values disclosed herein as being exemplary of a particular parameter. All such ranges are contemplated and within the scope of the present disclosure.

In the application, effective amounts are generally those amounts listed as the ranges or levels of ingredients in the descriptions, which follow hereto. Unless otherwise stated, all percentages, ratios, parts, and amounts used and described herein are by weight.

The phrase "free of ', "void of" or similar phrases if used herein means that the composition or article comprises 0% of the stated component, that is, the component has not been intentionally added. However, it will be appreciated that such components may incidentally form thereafter, under some circumstances, or such component may be incidentally present, e.g., as an incidental contaminant.

The phrase "substantially free of', "substantially void of' or similar phrases as used herein means that the composition or article preferably comprises 0% of the stated component, although it will be appreciated that very small concentrations may possibly be present, e.g., through incidental formation, contamination, or even by intentional addition. Such components may be present, if at all, in amounts of less than 1%, less than 0.5%, less than 0.25%, less than 0.1%, less than 0.05%, less than 0.01%, less than 0.005%, less than 0.001%, or less than 0.0001%. In some embodiments, the compositions or articles described herein may be free or substantially free from any specific components not mentioned within this specification.

Unless defined otherwise, all technical and scientific terms used herein have the same meaning as commonly understood by one of ordinary skill in the art to which the invention pertains. Although a number of methods and materials similar or equivalent to those described herein can be used in the practice of the present invention, the preferred materials and methods are described herein.

### II. Introduction

The present invention relates to compositions and methods for treatment of Long Covid, and similar post-viral or other post-infection conditions, e.g., particularly those that may result in residual toxins within the blood or bodily systems of the patient, where such toxins attack the red blood cells. For example, Applicant has found that in patients suffering from Long Covid, the red blood cell erythrocytes have been attacked, so as to decompose, as a result of a residual toxin associated with COVID-19 infection, within the body. Affected decomposed red blood cells are not able to effectively carry oxygen to the various tissues and organs, which is believed to be the root cause for may Long Covid symptoms. Applicant discovered that by treating the patient with a composition capable of neutralizing and flushing such toxins and decomposed "pulp" red blood cells from the body, the patient is able to recover their energy and health relatively quickly with the help of any medicaments needed for recovery of any particular organs affected by the Long Covid.

The contemplated treatment composition includes one or more alkaloid components, which are able to neutralize the residual alkaloid toxins that are the cause of the Long Covid symptoms, and the ongoing decomposition of red blood cells within Long Covid patients. In an embodiment, the treatment composition includes C₂₉H₄₀N₂O₄, C₉H₂N₂, and/or C₂₈H₃₈N₂O₄. In an embodiment, all 3 such alkaloid components are included. Additional components, particularly a source of iron (e.g., FePO₄) may also be present. A filler, such as calcium carbonate, or another pharmaceutically acceptable carrier may also be present.

Administration of the treatment composition can be provided as a single dose, one time, which is sufficient to neutralize the toxins within the blood stream and body, and allow the body to flush the neutralized toxins, and decomposed red blood cell "pulp" from the body, which then allows the body's natural immune system and any other conventional medicaments also administered, to aid the body in making a relatively quick recovery, e.g., within 10 days, typically within 2-5, 3-5, or 2-4 days.

While described principally in the context of treatment of Long Covid, the present treatment compositions and methods can also be used in treatment of other post viral and other post-infection conditions, particularly those that involve the presence of residual toxins within the blood cells and the blood stream or other bodily systems. Examples of such include, but are not limited to influenza, zika, herpes, and/or epstein-barr viruses.

The present invention also contemplates incorporation of the alkaloids, treatment compositions, or other components thereof, into drugs or other treatment compositions for treatment of Long Covid or other post-viral or other post-infection conditions, or for use in interference with the metabolism of COVID-19 or other viruses. For example, such compositions or described components thereof could be incorporated into other drugs or treatment compositions being developed for treatment of Long Covid or other post-infection conditions, particularly those that may involve residual hematoxins present within the patient suffering from such condition.

### III. Exemplary Compositions and Methods of Treatment

As noted, in an embodiment, the present treatment compositions include one or more alkaloid components. Such alkaloids may be toxin neutralizers, e.g., derived or isolated from ipecac, ipecacuanha (e.g., *Carapichea ipecacuanha* and/or *Cephaelis acuminata*) or related species.

In an embodiment, the treatment composition includes C₂₉H₄₀N₂O₄. In an example, the C₂₉H₄₀N₂O₄ may be emetine, a compound derived from ipecac root. Emitine has the structure shown below.

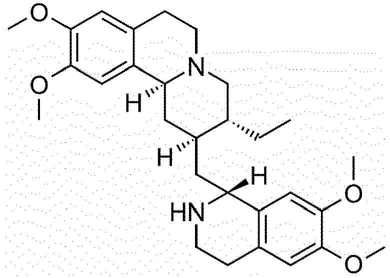

In an embodiment, the treatment composition, administered as a single dose, may include about 1/100,000 mg to about 1/1,000,000 mg, about 1/200,000 mg to about 1/750,000 mg or about 1/300,000 mg to about 1/750,000 mg of C₂₉H₄₀N₂O₄ (e.g., emetine). While administration of the treatment composition, one time only, as a single dose, may be preferred, it will be appreciated that in another embodiment, it may be possible to administer the full dosage of the treatment composition in multiple doses, e.g., divided into 2 doses, or 3 doses, 4 doses, 5 doses, or the like. Where such is the case, the single dose amount noted above could be divided by 2, 3, 4, 5, etc., as needed, so that the full dosage amount administered (e.g., over 2, 3, 4, 5, etc. doses) is as noted above for the single dosage example.

In an embodiment, the treatment composition includes C₂₈H₃₈N₂O₄. In an example, the C₂₈H₃₈N₂O₄ may be cephaeline, another alkaloid compound isolated from ipecacuanha. Cephaeline has the structure shown below.

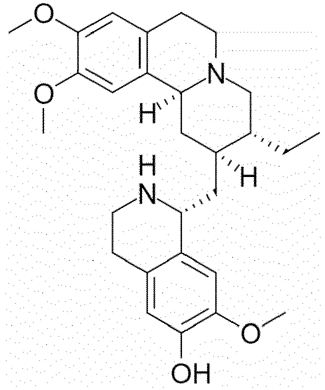

In an embodiment, the treatment composition, administered as a single dose, may include about 1/100,000 mg to about 1/1,000,000 mg, about 1/200,000 mg to about 1/750,000 mg or about 1/300,000 mg to about 1/750,000 mg of C₂₈H₃₈N₂O₄ (e.g., cephaeline). The dosage ratio of emetine to cephaeline may be about 1:5 to about 5:1, such as about 1:3 to about 3:1, or about 1:2 to about 2:1 (e.g., such as 1:1) (e.g., about 1/750,000 mg of emetine and about 1/750,000 mg of cephaeline). While administration of the treatment composition, one time only, as a single dose, may be preferred, it will be appreciated that in another embodiment, it may be possible to administer the full dosage of the treatment composition in multiple doses, e.g., divided into 2 doses, or 3 doses, 4 doses, 5 doses, or the like. Where such is the case, the single dose amount noted above could be divided by 2, 3, 4, 5, etc., as needed, so that the full dosage amount administered (e.g., over 2, 3, 4, 5, etc. doses) is as noted for the single dosage example.

In an embodiment, the treatment composition includes C₉H₂N₂. The C₉H₂N₂ may have the structure shown below.

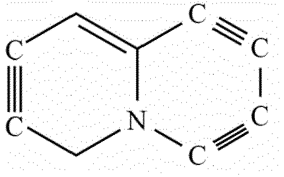

In an embodiment, 2, or all 3 of such alkaloid components are included. Additional components, particularly a source of iron (e.g., FePO₄) may also be present. A filler, such as calcium carbonate, or another pharmaceutically acceptable carrier may also be present.

In an embodiment, the treatment composition, administered as a single dose, may include about 1/50,000 mg to about 1/600,000 mg, about 1/60,000 mg to about 1/450,000 mg, about 1/60,000 mg to about 1/250,000 mg or about 1/90,000 mg to about 1/450,000 mg of C₉H₂N₂. The dosage ratio of C₉H₂N₂ to emetine may be about 2:1 to about 20:1, such as about 3:1 to about 15:1, about 5:1 to about 12:1 or about 6:1 to about 10:1 (e.g., about 1/750,000 mg of emetine and about 1/90,000 mg of C₉H₂N₂). While administration of the treatment composition, one time only, as a single dose, may be preferred, it will be appreciated that in another embodiment, it may be possible to administer the full dosage of the treatment composition in multiple doses, e.g., divided into 2 doses, or 3 doses, 4 doses, 5 doses, or the like. Where such is the case, the single dose amount noted above could be divided by 2, 3, 4, 5, etc., as needed, so that the full dosage amount administered (e.g., over 2, 3, 4, 5, etc. doses) is as noted for the single dosage example.

Where the full dosage is administered over 2, 3, 4, 5 or other number of smaller doses, such may be administered once per day, twice per day, 3 times per day, or the like.

As noted, in an embodiment, the treatment composition may also include a source of iron. FePO₄ is an exemplary iron source, although others are also possible, e.g., ferrous fumarate, ferrous sulfate, ferrous gluconate, and/or other iron salts.

In an embodiment, the treatment composition, administered as a single dose, may include about 0.5 to about 10 mg, about 1 to about 5 mg, about 1.5 to about 3 mg, or about 2 to about 3 mg of iron or the iron salt (e.g., FePO₄). While administration of the treatment composition, one time only, as a single dose, may be preferred, it will be appreciated that in another embodiment, it may be possible to administer the full dosage of the treatment composition in multiple doses, e.g., divided into 2 doses, or 3 doses, 4 doses, 5 doses, or the like. Where such is the case, the single dose amount noted above could be divided by 2, 3, 4, 5, etc., as needed, so that the full dosage amount administered (e.g., over 2, 3, 4, 5, etc. doses) is as noted for the single dosage example. In an embodiment, the treatment composition, administered as a single dose, may include about 1 to about 10 mg, about 2 to about 8 mg, or about 3 to about 6 mg of the CaCO₃ or other filler.

A filler or pharmaceutically acceptable carrier may also be provided, e.g., which may account for even a majority of the weight of the treatment composition. While calcium carbonate is an exemplary suitable filler, those of ordinary skill in the art will appreciate that numerous other filler materials, or pharmaceutically acceptable carrier materials are also or alternatively possible.

By way of example, a single dosage treatment composition may include about 1/750,000 mg of C₂₉H₄₀N₂O₄, about 1/90,000 mg of C₉H₂N₂, about 1/750,000 mg of C₂₈H₃₈N₂O₄, about 2.1 mg of the source of iron, and about 4.1 mg of CaCO₃. Such treatment composition may be referred to as ELENANT.

The components of the treatment composition may advantageously be configured to have a small molecular and/or particle size. For example, the components may be relatively small molecules (e.g., not large peptides, proteins, or the like, which may exhibit molecular weights of over 1000 g/mol). For example, the described alkaloid compounds have molecular weight values of no more than about 750 g/mol, or no more than about 500 g/mol. For example, emetine and cephaeline have molecular weight values of about 481 and about 467 g/mol, respectively. The molecular weight of C₉H₂N₂ is far lower, e.g., only about 138 g/mol. Such low molecular weight, small molecules are capable of penetrating the cell membranes of affected cells (e.g., red blood cells) in order to access and neutralize the target toxin materials, so that they can then be flushed from the body.

In addition to such components exhibiting low molecular weights, it can be important for the particle sizes of such components to similarly be on a micro or nano size scale, in order to allow the components to effectively penetrate the cell membrane, to access and neutralize the target toxin materials. For example, in an embodiment, average particle sizes of any of the described components may be very fine, e.g., less than about 100 µm, less than about 80 µm, less than about 50 µm, less than about 25 µm, less than about 10 µm, less than about 5 µm, less than about 3 µm, less than about 2 µm, less than about 1 µm, less than about 0.5 µm, less than about 0.1 µm less than about 0.05 µm, less than about 0.01 µm, less than about 0.005 µm, or less than about 0.001 µm.

Such fine particle sizes and molecular weights may also aid in ensuring that the composition does not induce vomiting upon ingestion (e.g., in contrast to some ipecac derived treatments used in the past, to induce vomiting in case of poisoning). In an embodiment, the treatment composition is not administered to induce vomiting (e.g., or for any conventional poisoning antigen treatment).

The described components are commercially available individually, for purchase at many pharmacies, e.g., such as those in Germany. The individual components may be combined and/or compounded together, e.g., for administration in a desired form (e.g., as a pill). It will be appreciated that any of a wide variety of delivery forms are possible, e.g., as a pill, as a powder, in liquid form (e.g., suspended or dissolved within a suitable carrier or solvent), or the like. Other forms of administration may also be possible, e.g., injection, inhalation, or otherwise.

It is important to note that the treatment composition is typically not administered while the patient is actively infected (e.g., testing positive) with COVID-19, as so long as the SARS-CoV-2 virus is active within the patient's body, even if the treatment composition were administered, the active viruses would continue to result in continued toxin production. Rather, the treatment composition is administered after the patient has recovered from their initial COVID-19 infection, e.g., once the patient no longer tests positive for COVID-19. In an embodiment, the patient may or may not be exhibiting symptoms of Long Covid. For example, the treatment composition could be administered shortly after a given patient has recovered from an initial COVID-19 infection, but before any Long Covid symptoms have in fact developed or manifested, e.g., to prevent onset of Long Covid. In another example, the patient may have exhibited Long Covid symptoms for a significant period of time (e.g., weeks or months or even a year or more) when the treatment composition is administered.

The treatment composition may be administered to individuals who have been vaccinated, as well as those who have not been vaccinated, e.g., so long as they have actually experienced a COVID-19 infection. It will thus be appreciated that the present treatment compositions can be used to treat a patient experiencing Long Covid symptoms, as well as to prevent a patient who recently recovered from an initial COVID-19 infection, from developing Long Covid.

The treatment composition advantageously results in clearance of Long Covid symptoms relatively quickly, e.g., within about 10 days, typically within about 3-5 days, about 2-5 days, or about 2-4 days.

### IV. Examples and Experimental Results

### Example 1

The Applicant, a female, aged 62, suffering from Long COVID for about 3 weeks, self-administered a single dose of ELENANT, as a poison control remedy. In addition to addressing any poison ingestion, the patient's Long Covid symptoms (lack of energy, dry cough) cleared up relatively quickly, within a period of 2 days.

### Example 2

A male patient, aged 28 (patient subject 188), suffering from Long COVID for about 8 weeks was administered a single dose of ELENANT. The patient's Long Covid symptoms (lack of energy, inflamed skin) cleared up relatively quickly, within a period of 7 days, with use of a medication for recovery of the inflamed skin.

### Example 3

Numerous male and female patients, of a wide range of ages, suffering from Long COVID, were each administered a single dose of ELENANT. The patients included individuals who had been vaccinated before contracting COVID-19, as well as individuals who had not been vaccinated before contracting COVID-19, All or nearly all treated patients experienced relief of their Long Covid symptoms, relatively quickly after being administered a single dose of ELENANT, within a period of 2-10 days.

### Analysis of Study on Long Covid

The length of the study considered a variety of patients over a period of over 19 months.

In total, there were 570 study participants. 46 participants were age 18 or younger. Of the remaining 524 adult study participants, 287 were women and 237 were men.

271 study participants reported active COVID-19 infection symptoms. 186 participants were found to be infected (i.e., tested positive) without exhibiting any significant infection symptoms. It was apparent in the results that since about August 2023 those who were infected with COVID-19, but exhibited no significant symptoms had increased.

All study participants infected with COVID-19 (including those who did not exhibit infection symptoms) had different amounts of cell disintegration substances in their body that disintegrate or functionally impair red blood cells.

113 study participants had no COVID-19 infection (i.e., tested negative). 22 study participants were tested for COVID-19, and they tested negative, although they exhibited symptoms of a regular cold.

334 study participants had been vaccinated against COVID-19 at least twice. Of those, 193 study participants had experienced a COVID-19 infection and had the cell disintegration substances as described herein in their cells.

236 study participants had not been vaccinated against COVID-19. Of those, 103 study participants had experienced a COVID-19 infection and had the cell disintegration substances as described herein in their cells.

The main symptom of red blood cell disintegration with 426 out of 457 of the study participants was a significant lack of energy. The symptoms mentioned with respect to such fatigue ranged from moderate fatigue to bedridden.
Other commonly reported symptoms reported included:

| | |
|---|---|
| - continuous slight cough | 92 study participants |
| - cramped muscles | 71 study participants |
| - heart / circulatory problems including | |
| high blood pressure | 56 study participants |
| - problems with shortness of breath | 38 study participants |
| - concentration problems | 38 study participants |
| - continuous cold symptoms | 28 study participants |
| - skin irritation | 21 study participants |
| - headaches | 18 study participants |
| - stomach / bowel problems | 4 study participants |

Other symptoms reported by at least a single participant included:
- diagnosed with Long Covid
- inflamed eyelids
- pressure in the face
- cannot get up
- red spots in front of the eyes
- pneumonia
- heavy legs, fluid retention in the legs
- inflamed joints
- panic attacks
- sleep problems

Two study participants exhibited heart oedema. They received the active agent ELENANT with subsequent clinical care for the heart oedema. In one male study participant the plaques/oedemas were aspirated from the heart. Administration of ELENANT substantially stopped further disintegration or functional impairment of red blood cells. The second male exhibiting a heart oedema refused clinical treatment because he felt fine. Both males recovered from the heart problems and are healthy.

The cell disintegration substances were neutralized:
- by 35 study participants within one hour (e.g., directly in the lab)
- by 303 study participants within 3 days (notification of being symptom-free)
- 91 study participants reported being Long Covid symptom free within 7 days
- 28 study participants had many additional diseases or complications such that they only overcame the Long Covid symptoms within about 10 days.

It is important to emphasize that the active agent ELENANT is believed to end the Long Covid symptoms exclusively through neutralization of the blood cell disintegration substances. All other diseases, conditions, or complications and their symptoms are not influenced by ELENANT, and must be treated separately.

### INDUSTRIAL APPLICABILITY

Following are sections in accordance with at least one embodiment of the present disclosure:

| | |
|---|---|
| A1. | A composition for use in treatment of Long Covid, or other post-viral or other post-infection conditions that result in a toxin induced thickened blood pulp condition, the composition comprising: |
| | C₂₉H₄₀N₂O₄; |
| | C₉H₂N₂; |
| | C₂₈H₃₈N₂O₄; and |
| | a source of iron. |
| A2. | The composition of section A1, further comprising CaCO₃. |
| A3. | The composition of any of sections A1-A2, wherein the source of iron comprises FePO₄. |
| A4. | The composition of any of sections 1-3, wherein the composition is administered in a single dose, wherein the single dose includes about 1/300,000 to about 1/750,000 mg of C₂₉H₄₀N₂O₄, about 1:60,000 to about 1/250,000 mg of C₉H₂N₂, about 1/300,000 to about 1/750,000 mg of C₂₈H₃₈N₂O₄, about 1 to about 5 mg of the source of iron, and about 1 to about 10 mg of CaCO₃. |
| B1. | A method for treatment of Long Covid, or other post-viral or other post-infection conditions that result in a toxin induced thickened blood pulp condition, the method comprising: |
| | providing a treatment composition comprising: |
| | C₂₉H₄₀N₂O₄; |
| | C₉H₂N₂; and/or |
| | C₂₈H₃₈N₂O₄; and |
| | optionally a source of iron; and |
| | administering the treatment composition to a patient, selected because of their exhibiting symptoms of Long Covid, or other post viral infection condition. |
| B2. | The method of section B1, wherein the treatment composition further comprises CaCO₃. |
| B3. | The method of any of sections B1-B2, wherein the treatment composition includes the source of iron, which comprises FePO₄. |
| B4. | The method of any of sections B1-B3, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, and about 1/300,000 mg to about 1/750,000 mg of C₂₈H₃₈N₂O₄. |
| B5. | The method of any of sections B1-B3, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, about 1/300,000 mg to about 1/750,000 mg of C₂₈H₃₈N₂O₄, and about 1 to about 5 mg of FePO₄. |
| B6. | The method of any of sections B1-B5, wherein the method is used to treat Long Covid. |
| B7. | The method of any of claim B1-B6, wherein the method is used to treat at least one of a post viral infection condition associated with influenza, zika, herpes, or epstein-barr viruses. |
| C 1. | A method for treatment of Long Covid, the method comprising: |
| | providing a treatment composition comprising: |
| | C₂₉H₄₀N₂O₄; |
| | C₉H₂N₂; |
| | C₂₈H₃₈N₂O₄; and |
| | optionally a source of iron; and |
| | administering the treatment composition to a patient, selected because of the patient's exhibition of symptoms of Long Covid. |
| C2. | The method of section C1, wherein the treatment composition further comprises CaCO₃. |
| C3. | The method of any of sections C1-C3, wherein the treatment composition comprises the source of iron, which comprises FePO₄. |
| C4. | The method of any of sections C1-14, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, about 1/300,000 mg to about 1/750,000 mg of C₂₈H₃₈N₂O₄, and about 1 to about 5 mg of FePO₄. |
| C5. | The method of any of sections C1-C5, wherein the treatment composition is administered as a single dose, the components thereof have a molecular or particle size sufficiently small so as to not induce vomiting, and the single dose administration to the patient is sufficient so that the patient's Long Covid symptoms are relieved within a period of about 10 days. |
| D1. | Use of a treatment composition in a drug or other treatment composition, for treatment of Long Covid, other post-viral, or other post-infection conditions, wherein the composition comprises: |
| | C₂₉H₄₀N₂O₄; |
| | C₉H₂N₂; and/or |
| | C₂₈H₃₈N₂O₄. |
| D2. | The use of claim D1, wherein the treatment composition further comprises a source of iron, which comprises FePO_{4.} |
| E1. | Use of a treatment composition to interfere with the metabolism of COVID-19 or other viruses, wherein the composition comprises: |
| | C₂₉H₄₀N₂O₄; |
| | C₉H₂N₂; and/or |
| | C₂₈H₃₈N₂O₄. |
| E2. | The use of claim E1, wherein the treatment composition further comprises a source of iron, which comprises FePO₄. |

Without departing from the spirit and scope of the invention, one of ordinary skill can make various changes and modifications to the invention to adapt it to various usages and conditions. As such, these changes and modifications are properly and intended to be, within the full range of equivalence of the following claims.

## Claims

1. A composition for use in treatment of Long Covid, or other post-viral or other post-infection conditions that result in a toxin induced thickened blood pulp condition, the composition comprising:
C₂₉H₄₀N₂O₄;
C₉H₂N₂;
C₂₈H₃₈N₂O₄;
and a source of iron.

2. The composition of claim 1, further comprising CaCO₃.

3. The composition of any of claims 1-2, wherein the source of iron comprises FePO₄.

4. The composition of any of claims 1-3, wherein the composition is administered in a single dose, wherein the single dose includes about 1/300,000 to about 1/750,000 mg of C₂₉H₄₀N₂O₄, about 1:60,000 to about 1/250,000 mg of C₉H₂N₂, about 1/300,000 to about 1/750,000 mg of C₂₈H₃₈N₂O₄, about 1 to about 5 mg of the source of iron, and about 1 to about 10 mg of CaCO₃.

5. A method for treatment of Long Covid, or other post-viral or other post-infection conditions that result in a toxin induced thickened blood pulp condition, the method comprising:
providing a treatment composition comprising:
C₂₉H₄₀N₂O₄;
C₉H₂N₂;
and/or
C₂₈H₃₈N₂O₄;
and
optionally a source of iron; and
administering the treatment composition to a patient, selected because of their exhibiting symptoms of Long Covid, or other post viral infection condition.

6. The method of claim 5, wherein the treatment composition further comprises CaCO₃.

7. The method of any of claims 5-6, wherein the treatment composition includes the source of iron, which comprises FePO₄.

8. The method of any of claims 5-7, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, and about 1/300,000 mg to about 1/750,000 mg of C₂₈H₃₈N₂O₄.

9. The method of any of claims 5-7, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, about 1/300,000 mg to about 1/750,000 mg of C₂₈H₃₈N₂O₄, and about 1 to about 5 mg of FePO₄.

10. The method of any of claims 5-9, wherein the method is used to treat Long Covid.

11. The method of any of claim 5-10, wherein the method is used to treat at least one of a post viral infection condition associated with influenza, zika, herpes, or epstein-barr viruses.

12. A method for treatment of Long Covid, the method comprising:
providing a treatment composition comprising:
C₂₉H₄₀N₂O₄;
C₉H₂N₂;
C₂₈H₃₈N₂O₄;
and
optionally a source of iron; and
administering the treatment composition to a patient, selected because of the patient's exhibition of symptoms of Long Covid.

13. The method of claim 12, wherein the treatment composition further comprises CaCO₃.

14. The method of any of claims 12-13, wherein the treatment composition comprises the source of iron, which comprises FePO₄.

15. The method of any of claims 12-14, wherein the treatment composition is administered in a single dose, wherein the single dose includes about 1/300,000 mg to about 1/750,000 mg of C₂₉H₄₀N₂O₄, about 1/60,000 mg to about 1/250,000 mg of C₉H₂N₂, about 1/300,000 mg to about 1/750,000 mg of C₂₈H₃₈N₂O₄, and about 1 to about 5 mg of FePO₄ and/or wherein the treatment composition is administered as a single dose, the components thereof have a molecular or particle size sufficiently small so as to not induce vomiting, and the single dose administration to the patient is sufficient so that the patient's Long Covid symptoms are relieved within a period of about 10 days.
